# EUROPEAN PATENT SPECIFICATION

(11) **EP 0 901 781 B1**
(45) Date of publication and mention of the grant of the patent: **19.12.2007**
(21) Application number: 98114701.0
(22) Date of filing: 30.01.1996
(51) Int. Cl.: A61F 13/15, A61F 13/00

(54) **Method for producing a continuous thermoplastic coating and articles constructed therefrom**
Verfahren zur Herstellung einer kontinuierlichen Beschichtung aus thermoplastischem Material, und daraus hergestellte Gegenstände
Procédé de production d'un revêtement thermoplastique continu et articles fabriqués à l'aide de celui-ci

(30) Priority: 23.02.1995 WO PCT/EP95/00665
(43) Date of publication of application: 17.03.1999
(62) Divisional of application: 96903949.4
(73) Proprietor: H.B. FULLER LICENSING & FINANCING, INC., St. Paul, Minnesota 55110-5132 (US)
(72) Inventor: Werenicz, Harald, 21391 Reppenstedt (DE); Wittkopf, Thomas, 21360 Vögelsen (DE); Voss, Gerhard, 21335 Lüneburg (DE); Remmers, Peter, 21039 Hamburg (DE); Katsaros, Mark G., Doloth, GA 30097 (US); Polance, Robert Gordon, Lino Lakes, MN 55014 (US); Kroll, Mark S., Arden Hills, MN 55112 (US)
(74) Representative: Maiwald, Walter

(56) References cited:
- EP-A- 0 187 728
- EP-A- 0 189 911
- EP-A- 0 295 694
- US-A- 4 627 847
- US-A- 4 692 161

## Description

### Field of the Invention

This invention relates to a non-contact coating method for producing a continuous coating and articles constructed therefrom. This invention further relates to a method for producing a textile material with a moisture-impermeable barrier layer and to a method for producing a moisture-absorbing article of hygiene which has such a barrier layer. This invention particularly relates to a textile material and hygienic disposable articles comprising a body fluid impermeable barrier layer which can be produced from said coating method. Preferably, the thermoplastic composition used in the coating method for producing the barrier layer exhibits certain rheological characteristics.

### Background of the Invention

For various applications, materials are required which are impermeable to liquids such as water and body fluids, but at the same time have a textile character which is as close as possible to materials without the impermeability characteristics. One example of such application is hygienic articles such as disposable diapers, feminine napkins, panty liners, surgical drapes, bed pads, and the like. Such hygienic articles often have a substantial absorption capacity. To ensure that the liquid which is to be absorbed does not reach articles of clothing, such hygienic articles customarily have a continuous layer of a body fluid impermeable film on their garment facing side. Since touching this film material is an unwelcome sensation for a number of users, it has been suggested to cover the outward side of the film with a thin layer of textile material so that the article has a textile feel also on its exterior surface. EP-A-O 187 728 teaches a disposable diaper with a barrier layer in the form of a plastics material film, typically consisting of a polyolefin, a polyacrylate, of PVC, nylon or other thermoplastic material. The barrier film is laminated, adhered or welded onto a thin nonwoven layer, which makes it necessary to prepare the film laminate off-line. As an alternative, it is suggested that the plastics material of the film is to be extrusion coated onto the nonwoven. The laminate, thus formed, is then used as the outermost material layer in such a way that the film layer is on the inside, so that the outer nonwoven layer provides the desired exterior texture. This production method is rather expensive. Highly polymeric plastic materials such as polyethylene, polypropylene, polyacrylate and so on, have low melt flow indices and can (if at all) only be processed into impermeable films on very sophisticated machinery. This would also be true for the suggested direct extrusion coating which does not appear to have been reduced to practice yet. Separately producing the film, with subsequent laminating onto the nonwoven, is even more inefficacious in view of the additional production step required.

EP 0 295 694, however, discloses a waterproof water-vapor permeable laminated structure obtained by extrusion laminating a thermoplastic resin layer on a porous base material. The thermoplastic resin layer to be employed in the laminate structure is selected tohave a melt viscosity of 5 x 10⁴ poise at a temperature lower than an extrusion temperature by 20°C.

US patents 4,692,161 and 4,627,847 teach a leakage waist barrier for the edge of an absorbent hygienic article provided by coating a hotmelt adhesive onto the edge area of a nonwoven sheet material. Depending on the actual type of application, this hotmelt adhesive can also serve a constructive function, in combination with its function as a barrier, in that it can adhere the nonwoven to other materials of the hygienic article. The hotmelt is to be coated in a conventional manner by slot nozzle coating, transfer coating, spray coating or other such methods. The above-mentioned US patents indicate that the hotmelt coating must have a minimum thickness of 25 µm, preferably at least 75 µm, so that a continuous closed barrier layer is achieved. Conventional slot nozzle coatings on uneven substrates such as nonwovens are typically done by keeping the slot nozzle in permanent contact with the substrate such that the nozzle lies on the substrate during the coating. It is unproblematic to coat hotmelt adhesives onto uneven substrates with slot nozzles or spray coating methods, provided that only a discontinuous coating is required such as for constructive applications wherein the coating weight of the hotmelt ranges from about 3 g/m² to about 1C g/m². If, however, a continuous layer is to be created, this can only be done using these customary coating methods if the coating weight of the hotmelt is greater than about 30 g/m².

Such high coating weights are expensive. Furthermore, direct coating with a slot nozzle provides substantial mechanical and thermal stresses on the coated substrates, especially since the slot nozzle is heated during coating. Therefore, very sensitive substrates such as nonwovens made of very fine or low melting point fibers can not always be coated with hotmelt from a slot nozzle in a customary manner without damaging the substrate.

Such problems can not be overcome when coating with heated coating rollers or spray coating with heated airstreams. The high coating weights of this prior art lead to increased stiffness of the coated substrate, thus impairing the textile character. Similar problems occur in the production of hygienic articles and in other areas, such as fabric production, wherein the resulting materials are required to exhibit liquid impermeability, especially body fluid impermeability, with textile character which is as unimpaired as possible. This is especially pertinent for improving the comfort of the user. Presently, in such technical fields, production methods utilizing preformed laminated films are preferred.

Therefore, there remains a need for a non-contact method capable of producing a continuous coating layer having low coating weights.

### Summary of the Invention

The applicants have found a coating method that overcomes the aforementioned problems. The coating method employs a noncontact application wherein a thermoplastic composition is thermally made flowable and released from a coating device onto a substrate. The thermoplastic composition is thus coated onto the substrate without contact between said coating device and said substrate. Preferably, a liquid-impermeable, especially a body fluid impermeable, barrier wherein the textile character is not substantially impaired is produced. Since the method employs low coating weights of the thermoplastic composition, it eliminates the economic disadvantages of current methods as well as improves the tactile quality of the resulting article. Additionally, the method is suitable for coating a variety of heat sensitive materials. Preferably, the substrate is a "textile material" which in the context of this invention means not only a woven material made of yarn, but also any material made from fibers such as nonwoven, as well as nonwoven composites and the like, which materials play a major role in the area of hygienic article production. Since the coating device and substrate do not contact each ether, the mechanical stresses on the substrate are much less than prior art methods.

For heat sensitive substrate, the thermoplastic composition is preferably coated at temperatures of less than 125°C, even more preferably less than 110°C, to reduce the heat-induced stresses on the substrates being coated. This is advantageous for coating and mutually bonding thermally sensitive substrates. The thermoplastic composition preferably exhibits certain rheological characteristics such that the complex viscosity at high shear rates (1,000 rad/sec) is less than about 500 poise and the complex viscosity at low shear rates (1 rad/sec) is between about 100 and about 1,000 poise. Some neat thermoplastic resins, such as typical film grade polyolefines, may be suitable for the method of the present invention. However, compounded hotmelt adhesives are preferred due to the ability to independently control the visco-elastic properties, open time, etc. Compounded hotmelt are also advantageous to insure adequate adhesion to the carrier substrate or for delayed detackification of the coating after adherence to the substrate.

The resulting coating produced from said method is useful for a variety of applications wherein a consistent continuous coating is desired. Coating weights of less than 30 g/m² of the thermoplastic composition are preferred to reduce expenditure and improve the tactile quality of disposable hygienic articles. However, coating weights higher than 30 g/m² may be useful for other applications wherein reducing the mechanical and heat-induced stresses is of primary importance.

The resulting coating is preferable for producing a body fluid impermeable barrier layer in a disposable hygienic article having improved exterior tactile quality. The coating method is particularly advantageous for manufacturing as it employs fewer production steps than prior art coating methods. Improving productivity as well as reducing the coating weight mass per area results in coatings and corresponding articles that are less expensive than prior art.

The disposable article as described herein has at least one first layer wherein said first layer is a permeable substrate and at least one second layer wherein said second layer is a barrier coating produced from the coating method described above.

### Brief Description of the Drawing

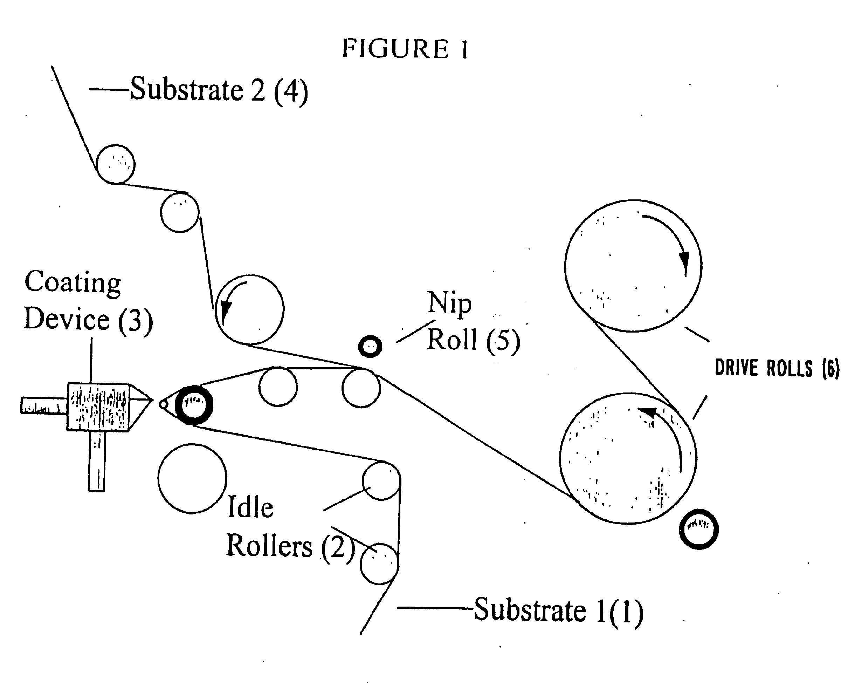

Figure 1 depicts the method of the present invention wherein a continuous thermoplastic coating is formed and adhered to a carrier substrate.

### Detailed Description of the Drawing

Substrate 1 (1) travels past a series of idle rollers (2) to ensure the web is in proper alignment prior to approaching the coating device (3). At start up the coating device is initially in contact with the substrate to adhere the leading edge of the coating to the substrate. As Substrate 1 is advanced by the drive rolls (6), the coating device is removed from Substrate 1 at a distance most often ranging from about 0.5 mm to about 20 mm, depending on the properties of the thermoplastic composition being coating. Substrate 2 (4) is optionally adhered to the coating surface by means of a nip roll (5). In the preferred embodiment, Substrate 2 may be any substrate present in a hygienic article such as an absorbent, elastomeric strands or webs, tissue, films, coverstock materials such as nonwoven or perforated polyethylene, as well as any material that is not necessarily in the form of a web such as superabsorbent polymer.

### Detailed Description of the Invention

In the method of the present invention, a melted hotmelt adhesive, preferably substantially air-free, is released from a coating or release device in such a way that it exits the device as a continuous film. A typical example for this is a slot nozzle, as it has previously been used for coating in direct contact with a substrate. Thus, melt coating devices which are already at hand can be reset for use according to the invention in that the slot nozzle is lifted off the substrate and is adjusted to have a suitable distance from the substrate. When the viscous but flowable molten adhesive leaves the coating device, it does not contact the substrate immediately, but rather travels for a distance as a continuous coating film suspended above the substrate without touching either the device cr the substrate. The distance between the coating device and the substrate ranges from about 0.5 mm to about 20 mm. It is possible that at suitable machine speed settings, and with specific adhesives or other coating materials, the distance can be even greater than 20 mm. The distance is largely dictated by the viscosity and open time of the thermoplastic composition being coated. It is surmised that the thermoplastic composition cools sufficiently in its suspended state such that it has built in viscosity and cohesive strength to the extent that any filaments or fibers present on the substrate surface cannot penetrate the coating, yet the thermoplastic composition is molten or soft enough to adequately adhere to the substrate.

It has been shown to be especially advantageous, that the coating later contacts the substrate in a substantially horizontal direction rather than in a vertical direction. To realize this advantage, a roller can be provided in the path of movement of the substrate to give the substrate a substantially vertical, upward direction, as the substrate passes the coating device.

Additionally, the coating device, such as a slot nozzle, can be provided substantially horizontally beside the roller so that the coating travels from the side towards the surface of the substrate.

The diameter of the coating role is preferably about 15 mm to about 50 mm in diameter with the nozzle slightly above the center of the coating roll such that the angle at which the thermoplastic coating contacts the substrate is less than about 60° as the substrate is moving away form the nozzle. The coating head is adjusted by one of ordinary skill in the art to optimize for even flow and distribution of the thermoplastic coating over the entire width of the application.

Thereafter, the sufficiently cooled coating contacts the substrate surface and adheres to the surface without deeply penetrating into the substrate. When the substrate is preferably a textile material such as a nonwoven, the thus produced material comprises the textile substrate layer and a coating, preferably a hotmelt barrier layer. If the thermoplastic coating is of such a composition that it substantially detackifies after sufficient cooling, the laminate of the coated substrate, thus formed, can be rolled up and stored. The laminate can then be used at some later time e.g. as a body fluid impermeable backsheet having improved tactile quality in a disposable hygienic article. The laminate can be bonded by any suitable bonding technique including ultrasonic bending, heat welding, or more commonly adhesive bonding.

Preferably, the coating of the barrier layer is done "inline" immediately before any further processing of the this produced coated textile laminate. In such a case, the surface of the barrier layer which is pointing away from the substrate and is still sufficiently tacky can be used for a constructive adhesion step and therefore can also serve to bond the coated textile material to other elements of a hygienic article. Other elements that could be simultaneously bonded in this manner during the formation of the barrier layer include absorbent, superabsorbent polymer, elastomeric strands or webs, tissue, films, as well as various permeable coverstock materials such as nonwoven or perforated films.

Since the hotmelt coating can be provided at extremely low temperatures, materials can also be provided with barrier layers which are too sensitive mechanically and/or thermally for customary coating methods.

Such sensitive materials include low gauge polyethylene materials, low basis weight nonwovens and the like.

A substantial advantage of the invention is that continuous, sufficiently impermeable barrier layers can be made from hotmelts at very low coating weights. Even with customary commercially available hotmelts, closed barrier layers can be produced at coating weight of not more than 30 g/m², and generally, it is easily possible to achieve coating weights between 10 g/m² and 20 g/m² and most preferably less than 10 g/m². As previously stated, the prior art coating of hotmelts according to customary methods for forming edge leakage barriers, as in US patent 4,692,161, requires area weights of about 70 g/m² to create the preferred film thickness of around 75 µm. At thickness of 25 µm, the suggested minimum according to this art, the contact-coated layer is perforated by substrate fibre, and is not closed.

The very thin barrier layers which can be produced according to the invention do not only contribute to the economical advantages of the inventive method, but also make it possible to achieve a very much reduced stiffness of the material, which thus comes much closer, in its properties, to a textile material which is not provided with a barrier layer at all.

### The Thermoplastic Composition

As previously mentioned, uncompounded thermoplastic materials such as polyolefins, especially polyethylene, polypropylene, amorphous polyolefins such as Vestoplast 703® (Hüls) and the like, may be suitable thermoplastic materials for the coating method of the present invention. However, hotmelt adhesives are preferred due to the ability to independently tailor the visco-elastic properties, open time, tack, and various other properties. Hotmelt adhesives commonly have melt flow indices required for such processing already at very low temperatures. Typical hotmelts are fluid enough for such processing at temperatures ranging from about 60°C to 110°C.

More preferably, the thermoplastic composition exhibits certain rheological characteristics such that a continuous, especially a body fluid impermeable coating can be produced at coating weights of less than about 30 g/m². In general, the rheological properties preferably fall within a rheological window wherein the complex viscosity at high shear rates (1,000 rad/sec) is less than about 500 poise and the complex viscosity at low shear rates (< 1 rad/sec) is between about 100 and about 1,000 poise. In other words, preferable thermoplastic compositions exhibit Newtonian regions at low shear rates and shear thinning at higher shear rates. Thermoplastic compositions having wide windows of application are those in which the composition exhibits the appropriate rheological properties at a variety of application settings, particularly low temperatures. Narrow application windows are those in which the rheological parameters are only met under very specific conditions. Amorphous polyolefins based hotmelt adhesives such as Lunatack® D-8370 (H.B. Fuller Company) tend to exhibit relatively flat viscosity curves at low shear rates.(less than bout 10 rad/sec) and consequently relatively wide application windows. Block copolymer based hotmelt adhesives tend to exhibit particularly steep viscosity profiles causing very narrow application windows.

Data generated that supports this rheological window is depicted in Table I. The test procedures used to determine the rheological data are described in detail hereinafter.

The applicants surmise that the high shear information relates to the processing conditions at the slot die exit. A composition with too high of a complex viscosity at 1,000 radians/sec would require excessive pump pressure to exit the coating device. A die with a shim gap larger than 3 mm could be used to process these materials but a higher coating weight may result.

The low shear information relates to the settling of the coating on the substrate during the time it is suspended above the substrate. If the low shear value is too high, the coating may not adhere adequately to the substrate and/or the thermoplastic composition builds up at the nozzle causing a streaked, discontinuous coating. If the low shear viscosity is too low, the coating may seep into the substrate, causing poor barrier properties.

Extensional viscosity, which was not measured can also strongly influence the melt strength. Higher levels of branching or the addition of a small concentration of a high molecular weight material can strongly influence the melt strength. More preferred, are compositions that meet the target rheological parameters at low application temperatures of less than about 125°C, most preferably less than about 110°C.

Accordingly, many known hotmelt adhesive compositions are well suited for use in the coating method of this invention. Hotmelt adhesives typically comprise at least one thermoplastic polymer, at least one plasticizer and at least one tackifying resin. Preferably, such suitable hotmelts comprise up to 40% by weight of thermoplastic polymer, up to 40% by weight of a plasticizer and up to 70% by weight of tackifying resin.

With respect to the thermoplastic polymer, atactic polyalphaolefins such as Vestoplast® 708 (Hüls) and synthetic rubbers such as S-EB-S block copolymers have been found to be especially suited, particularly those as used in hotmelt adhesives such as Lunatack® D-3964 (H.B. Fuller). Further, however, also esther thermoplastic polymers are suitable, such as ethylene-vinylacetate copolymers or other synthetic rubbers as available in commerce under the tradenames Kraton®, Solprene®, and Stereon®.

Plasticizers and tackifying resins used in hotmelt adhesives are known. Oils such as naphthenic oils are preferred plasticizers. As for tackifying resins, those resins already known for such purposes are generally suitable, especially hydrocarbon resins, ester resins and other such compatible resins. The components are mixed and processed in a known manner to prepare the hotmelts which can be used according to this invention.

With suitable hotmelts, such as those described in DE-A-41 21 716, it is also possible to make materials which are impermeable to liquid water, yet water vapor permeable rendering the coating "breathable".

In addition to commonly known hotmelt adhesives, thermoplastic compositions comprising a water soluble, saline (body fluid) insoluble copolyester such as Eastman AQ 1350®, commercially available from Eastman, are also particularly useful for creating barrier films that are impervious to body fluid, yet readily water soluble. This feature is of particular interest for creating flushable and compostable disposable hygienic products. Furthermore, there may be applications wherein water permeability is desired. Accordingly, this coating method may also be suitable for coating water permeable, water soluble and/or biodegradable thermoplastic materials.

Hereinafter, the invention will be further depicted by the following non-limiting examples.

### Embodiment example 1:

Several hotmelts which slightly differ from each other in composition were formulated in the following composition ranges:
20 - 25% naphthenic oil
30 - 40% atactic polyolefin(s)
35 - 45% hydrocarbon resin

### Embodiment example 2:

Several hotmelts were formulated within the following range limits:
15 - 20% SIS-block copolymer
15 - 25% naphthenic oil
50 - 65% ester resin

### Embodiment example 3:

As a commercially available hotmelt adhesive, the Ilunatack D 8370" product was used, which is available from H. 3. Fuller GmbH. This is a hot-melt adhesive comprising about 35% polyolefine, about 40% hydrocarbon resin with a cyclopentadiene component, about 15% polyisobutylene and about 10% naphthenic oil.

### Testing Procedure

The hotmelts according to embodiment examples 1 through 3 were placed in a customary processing machine provided with a slot nozzle such as Nordson EP 51. The slot nozzle was provided horizontally facing a roller over which a polypropylene nonwoven was led in an upward direction. The distance between the slot nozzle and the substrate was 2 mm, at a nozzle slot length of 70 mm. The web speed of the nonwoven was 25 m/min. At a system pressure of about 53 bar and a release temperature of the hot-melt of approximately 100°C, the hotmelt was coated onto the substrate, where it formed a closed barrier layer. Immediately thereafter, the thus coated substrate was adhered to a customary absorptive body (tissue). In each case, a reliable adhesive bond between substrate and tissue was provided, and in each case, the hotmelt barrier layer formed between the tissue and the substrate was found to be completely liquid-impermeable. Processing was without any problems. The coating weight was an average of 21 g/m². At corresponding fine adjustment of release temperature of hotmelt, system pressure, distance between slot nozzle and substrate, machine speed etc. etc., it was systematically possible to form water-tight closed barrier layers at area weights of less than 20 g/m² on this substrate.

### Examples 4-16:

Table 1 depicts rheological data on Examples 4 through 16.

Column 2 of Table 1 depicts the reference temperature for the rheological parameters as well as the coating application temperature for each sample. Table 2 depicts the chemical description of examples 4 through 9 as well as the coating parameters for those examples in which a continuous coating resulted. A consistent continuous coating was not able to be produced with Samples 4 through 9 at the temperature indicated in Column 2. The applicants surmise that the inability to produce a continuous coating is due to the complex viscosity being greater than about 1000 poise at about 1 rad/sec. By comparing examples 5 with 14 and 4 with 10, the complex viscosity at 1 rad/sec can be forced into the rheological window by increasing the temperature. By comparing example 7 with 16, the applicants have demonstrated the relatively narrow rheological window of Lunatack® D-3964. At 90°C D-3964 exhibits too high of a complex viscosity at 1 rad/sec. At 110°C, D-3964 exhibits to low of a complex viscosity at 1 rad/sec, causing the material to soak into the substrate. The applicants surmise a temperature exists between 90°C and 110°C wherein D-3964 would produce a continuous coating. However, a thermoplastic composition exhibiting such a narrow theological window would have little chance of commercial success.

Example 14 exhibits the utility of blending a thermoplastic composition that does not meet the rheological window with another material such that the resulting composition is useful for producing a continuous coating. In this particular example, since D-3964 exhibits too low of a complex viscosity at 1 rad/sec, it is blended with a material to raise the complex viscosity at 1 rad/sec such that the blend exhibits the preferable rheological properties. Alternatively, examples exhibiting too high of a complex viscosity at 1 rad/sec, such as examples 4 through 9 can be blended with compatible materials to lower the complex viscosity such that the blended material may be coated at the preferable application temperature of less than 125°C.

Examples 4 through 16 were tested in a similar manner as Examples 1 - 3. The application conditions and rheological data of the adhesive compositions are depicted in Table 1. A system pressure ranging from about 40 to about 65 bar was obtained during coating of examples 10 through 16.

The rheological data was generated from a dynamic mechanical spectrometer such as a Rheometric Scientific RDS 7700 (10, 000 g/cm transducer, 10 g/cm - <10,000 g/cm torque) . A master curve of G' (shear storage modulus), G'' (shear loss modulus) and complex viscosity as a function of frequency was obtained through time temperature superposition. During testing the sample was loaded at the upper test temperature between 50 mm diameter parallel plate discs with a 1 to 2 mm gap. After allowing the sample temperature to stabilize for at least about 10 minutes, a frequency sweep was performed from about 0.1 to about 100 radians per second. Upon the completion of the frequency sweep, the sample temperature was lowered to the next temperature and the procedure repeated. The strain amplitude was adjusted to improve the resolution and ranged from about 20% to about 40%. After the frequency sweep was completed at the final, lowest temperature, time-temperature superposition was used to overlay the data into a single master curve at the application temperature. If the actual coating temperature was not one of the actual temperatures tested, the Williams, Landel, Ferry (WLF) (Ferry, J.D. Viscoelastic Properties of Polymers, 3rd Ed., Wiley: NY, 1980) equation was used to obtain the master curve.

**Table 1**

| Example | Temp. (°C) | Complex Viscosity 1 rad/sec (poise) | Complex Viscosity 10³ rad/sec (poise) | G' 1 rad/sec (dynes/cm²) | Crossover Tan Frequency (rad/sec) | delta @ 1 rad/sec | slope=[Visc @ 1 rad/Sec]/[Visc@1000 rad/Sec] | Continuous Coating Formed yes/no |
|---|---|---|---|---|---|---|---|---|
| 4 | 125 | 15000 | 100 | 10000 | 1 | 1 | 150 | no |
| 5 | 90 | 10000 | 300 | 3000 | 300 | 3 | 33 | no |
| 6 | 120 | 4500 | 1500 | 300 | 1000 | 30 | 4.5 | no |
| 1 | 90 | 3000 | 100 | 400 | 50 | 7 | 30 | not tested |
| 8 | 110 | 2000 | 500 | 700 | 10000 | 3 | 4 | no |
| 9 | 140 | 1000 | 500 | 50 | >1000 | 70 | 2 | no |
| 10 | 160 | 200 | 200 | 5 | >1000 | 4 | 1.25 | yes |
| 11 | 125 | 800 | 100 | 5000 | 20 | 1 | 8 | yes |
| 12 | 125 | 800 | 100 | 100 | 1000 | 10 | 8 | yes |
| 13 | 125 | 300 | 50 | 200 | 1 | 1 | 6 | not tested |
| 14 | 110 | 300 | 50 | 20 | 7000 | 20 | 6 | yes |
| 15 | 128 | 100 | 80 | 10 | 1000 | 10 | 1.25 | yes |
| 16 | 110 | 100 | 3.5 | 8 | 100 | 25 | 28 | 110 |

**Table 2**

| Example | Tradename(s) | Chemical Description | Coating Weight (GSM) | Speed M/MIN | Permeability cm³ of H₂O pressure |
|---|---|---|---|---|---|
| 4 | 347-BD-19 (H.B. Fuller) | atactic polyolefin hotmelt adhesive (IIMA) | | | |
| 5 | D-3964 + 10% Vestoplast® 750 | SEBS block copolymer/hydrocarbon resin/napthenic oil HMA + atactic polyolefin | | | |
| 6 | Eastman AQ® 1350 | water dispersible copolyester (WO 95/18191) | | | |
| 7 | D-3964 | SEBS block copolymer/hydrocarbon resin/napthenic oil HMA | | | |
| 8 | NP-2085 (HBF) | urethane | | | |
| 9 | Eastman AQ® 1350 | See Example 9 | | | |
| 10 | Eastman AQ® 1350 | See Example 9 | | | |
| 11 | Vestoplast® 703 (Huls) | atactic polyolefin | 22 | 30 | - |
| 12 | 347-BD-33 (HBF) | atactic polyolefin HMA | 10 | 30 | 100 |
| 13 | Vestoplast® 703 + 10% Paraflint 114 | atactic polyolefin + Fischer Tropsch wax | | | |
| 14 | D-3964 + 10% Vestoplast 750 | SEBS block copolymer/hydrocarbon resin/napthenic oil I IIMA atactic polyolefin | 9-11 | 34 | 50 |
| 15 | D-8270 | atactic polyolefin IIMA | 12 | 30 | 46 |
| 16 | D-3964 | See Example 10 | 8 | 30 | - |
| 17 | Vestosplast® 750 10 % Wax | See Example 13 | 15-16 | 30 | - |

## Claims

1. A method of coating a thermoplastic composition onto a substrate, said method comprising the steps of:
a) making a thermoplastic composition flowable;
b) advancing a substrate along a path;
c) dispensing a continuous film of said thermoplastic composition from a coating device at a coating temperature wherein the complex viscosity of the thermoplastic composition is less than about 500 poise at about 1000 radians/second and ranges from about 100 poise to about 1000 poise at about 1 radian/second;
d) suspending said film between said coating device and said substrate;
e) contacting said film with said advancing substrate.

2. The method of Claim 1, wherein said thermoplastic composition comprises a hot-melt adhesive composition.

3. The method of Claim 2 further comprising suspending said continuous film such that said film builds in viscosity and cohesive strength such that any fibers or filaments present in the substrate do not penetrate said continuous film.

4. The method according to claim 1 to 3, wherein said substrate is selected from the group consisting of woven-, nonwoven textile material, heat sensitive materials and plastic materials.

5. The method according to any of claims 1 to 4, **characterized in that** said coating device is disposed at a distance from said substrate preferably of at least about 0.5 mm, and more preferably not more than about 20 mm and wherein the coating device is preferably a slot nozzle.

6. The method according to any one of claims 1 to 5, **characterized in that** the coating device is provided in a part of the path of movement of the substrate wherein said path of movement is directed substantially vertically.

7. The method according to any one of claims 1 to 6, **characterized in that** the thermoplastic composition or the hot-melt adhesive composition is coated onto the substrate such that the coating weight is not more than about 30 g/m², preferably not more than about 20 g/m² and especially preferred about 10 g/m² or less.

8. The method according to any one of claims 1 to 7, **characterized in that** the thermoplastic composition or the hot-melt adhesive composition is released from the coating device at a temperature of less than about 125°C, preferably less than about 110°C.

9. The method according to any one of claims 1 to 7, wherein the hot-melt adhesive is released from the coating device at a temperature less than about 160°C.

10. A method for producing a moisture-absorbing hygienic article comprising at least one structural element of absorbent material, at least one moisture-impermeable, in case water vapor-permeable inner barrier layer substantially covering the absorbent element and at least one outer layer of textile material which is bonded to the barrier layer on one face, **characterized in that** for forming the inner barrier layer, a thermoplastic composition or hot-melt adhesive composition is coated according to any one of claims 1 to 9 as a continuous closed film from a coating device onto the textile material of the outer layer without the coating device contacting the substrate.

11. The method according to claim 10, **characterized in that** the thermoplastic composition, preferably formed by a hot-melt adhesive film, is coated onto the substrate with an area weight not exceeding 30 g/m², preferably not exceeding 20 g/m² and especially preferred less than 10 g/m².

12. The method according to claim 10 or 11, **characterized in that** the thermoplastic composition forming the barrier layer is coated onto the textile material and said material is thereafter combined with the material forming the absorbent element so that the hot-melt is only adhered at the surfaces of the textile material and the absorbent element and thus bonds the textile material and the element to each other.

13. The method according to claim 12, **characterized in that** the textile material and the absorbent element are combined and bonded "inline" immediately after the coating of the hot-melt.

14. A method of coating, wherein a thermoplastic composition or a hot melt adhesive composition which has been thermally made flowable, is coated from a coating device onto a substrate, **characterized in that** said composition is released from said coating device at a temperature of less than 125°C, preferably less than 110°C as a continuous coating without contact between said coating device and said substrate, said composition having a complex viscosity of less than 500 poise at 1000 radians/seconds at the coating temperature and a complex viscosity ranging from 100 poise to 1000 poise at 1 radian/second at the coating temperature, and is subsequently disposed upon the surface of said substrate.

15. A disposable hygienic article, comprising at least one first layer, which is a body fluid permeable substrate, and at least one second layer which is a body fluid impermeable barrier layer formed by the method of any one of claims 10 through 14.

## Patentansprüche

1. Verfahren zum Aufbringen einer thermoplastischen Zusammensetzung auf ein Substrat, wobei das Verfahren die Schritte umfasst:
a) Fließfähig machen einer thermoplastischen Zusammensetzung;
b) Befördern eines Substrates entlang eines Weges;
d) Verteilen eines kontinuierlichen Films der thermoplastischen Zusammensetzung von einer Beschichtungsvorrichtung bei einer Beschichtungstemperatur, wobei die komplexe Viskosität der thermoplastischen Zusammensetzung weniger als etwa 500 Poise bei etwa 1000 Radiant/sek. beträgt und von etwa 100 Poise bis etwa 1000 Poise bei etwa 1 Radiant/sek. reicht;
d) Suspendieren des Films zwischen der Beschichtungsvorrichtung und dem Substrat;
e) Kontaktieren des Films mit dem beförderten Substrat.

2. Verfahren gemäß Anspruch 1, wobei die thermoplastische Zusammensetzung eine Schmelzklebezusammensetzung umfasst.

3. Verfahren gemäß Anspruch 2, weiterhin umfassend Suspendieren des kontinuierlichen Films, so dass der Film an Viskosität und Kohäsionskraft gewinnt, so dass jegliche in dem Substrat vorhandenen Fasern oder Filamente nicht in den kontinuierlichen Film eindringen.

4. Verfahren gemäß Anspruch 1 bis 3, wobei das Substrat ausgewählt ist aus der Gruppe, bestehend aus gewebten Textilmaterialien, Vliestextilmaterialien, wärmeempfindlichen Materialien und Kunststoffmaterialien.

5. Verfahren gemäß einem der Ansprüche 1 bis 4, **dadurch gekennzeichnet, dass** die Beschichtungsvorrichtung in einem Abstand zu dem Substrat von bevorzugt mindestens etwa 0,5 mm und stärker bevorzugt nicht mehr als etwa 20 mm angeordnet ist, und wobei die Beschichtungsvorrichtung vorzugsweise eine Schlitzdüse ist.

6. Verfahren gemäß einem der Ansprüche 1 bis 5, **dadurch gekennzeichnet, dass** die Beschichtungsvorrichtung in einem Teil des Beförderungsweges des Substrates bereitgestellt wird, in welchem der Bewegungsweg im Wesentlichen vertikal gerichtet ist.

7. Verfahren gemäß einem der Ansprüche 1 bis 6, **dadurch gekennzeichnet, dass** die thermoplastische Zusammensetzung oder die Schmelzklebezusammensetzung so auf das Substrat aufgebracht wird, dass das Beschichtungsgewicht nicht mehr als etwa 30 g/m², vorzugsweise nicht mehr als etwa 20 g/m² und besonders bevorzugt etwa 10 g/m² oder weniger beträgt.

8. Verfahren gemäß einem der Ansprüche 1 bis 7, **dadurch gekennzeichnet, dass** die thermoplastische Zusammensetzung oder die Schmelzklebezusammensetzung von der Beschichtungsvorrichtung bei einer Temperatur von weniger als etwa 125 °C, vorzugsweise weniger als etwa 110 °C abgegeben wird.

9. Verfahren gemäß einem der Ansprüche 1 bis 7, wobei der Schmelzkleber von der Beschichtungsvorrichtung bei einer Temperatur von weniger als etwa 160 °C abgegeben wird.

10. Verfahren zur Herstellung eines Feuchtigkeit absorbierenden Hygieneartikels, umfassend mindestens ein Strukturelement aus absorbierendem Material, mindestens eine feuchtigkeitsundurchlässige, gegebenenfalls wasserdampfdurchlässige innere Barriereschicht, welche das Absorptionselement im Wesentlichen bedeckt, und mindestens eine äußere Schicht aus Textilmaterial, die auf einer Seite an die Barriereschicht gebunden ist, **dadurch gekennzeichnet, dass** zum Bilden der inneren Barriereschicht eine thermoplastische Zusammensetzung oder Schmelzklebezusammenfassung nach einem der Anprüche 1 bis 9 als ein kontinuierlicher geschlossener Film von einer Beschichtungsvorrichtung auf das Textilmaterial der äußeren Schicht aufgebracht wird, ohne dass die Beschichtungsvorrichtung das Substrat kontaktiert.

11. Verfahren gemäß Anspruch 10, **dadurch gekennzeichnet, dass** die thermoplastische Zusammensetzung, vorzugsweise von einem Schmelzklebefilm gebildet, auf eine Seite des Substrates mit einem Flächengewicht beschichtet wird, welches 30 g/m² nicht übersteigt, vorzugsweise 20 g/m² nicht übersteigt und besonders bevorzugt weniger als 10 g/m².

12. Verfahren gemäß Anspruch 10 oder 11, **dadurch gekennzeichnet, dass** die thermoplastische Zusammensetzung, die die Barriereschicht bildet, auf das Textilmaterial aufgebracht wird und das Material danach mit dem Material kombiniert wird, das das Absorptionselement bildet, so dass die Schmelze nur an den Oberflächen des Textilmaterials und des Absorptionselementes haftet und damit das Textilmaterial und das Element aneinander bindet.

13. Verfahren gemäß Anspruch 12, **dadurch gekennzeichnet, dass** das Textilmaterial und das Absorptionselement unmittelbar nach dem Aufbringen der Schmelze kombiniert und "inline" gebunden werden.

14. Beschichtungsverfahren, wobei eine thermoplastische Zusammensetzung oder eine Schmelzklebezusammensetzung, welche thermisch fließfähig gemacht wurde, von einer Beschichtungsvorrichtung auf ein Substrat aufgebracht wird, **dadurch gekennzeichnet, dass** die Zusammensetzung bei einer Temperatur von weniger als 125 °C, vorzugsweise weniger als 110 °C als eine kontinuierliche Beschichtung von der Beschichtungsvorrichtung abgegeben wird, ohne Kontakt zwischen der Beschichtungsvorrichtung und dem Substrat, wobei die Zusammensetzung eine komplexe Viskosität von weniger als 500 Poise bei 1000 Radiant/sek. bei der Beschichtungstemperatur und eine komplexe Viskosität, die von 100 Poise bis 1000 Poise bei 1 Radiant/sek. bei der Beschichtungstemperatur erreicht, und nachfolgend auf der Oberfläche des Substrates verteilt wird.

15. Wegwerfhygieneartikel, umfassend mindestens eine erste Schicht, welches ein für Körperflüssigkeiten durchlässiges Substrat ist, und mindestens eine zweite Schicht, welches eine für Körperflüssigkeiten undurchlässige Barriereschicht ist, hergestellt mit dem Verfahren nach einem der Ansprüche 1 bis 14.

## Revendications

1. Procédé de revêtement d'une composition thermoplastique sur un substrat, ledit procédé comportant les étapes consistant à :
a) amener une composition thermoplastique à pouvoir s'écouler,
b) faire avancer un substrat le long d'un trajet,
c) distribuer un film continu de ladite composition thermoplastique à partir d'un dispositif de revêtement à une température de revêtement à laquelle la viscosité complexe de la composition thermoplastique est inférieure à environ 500 poises à environ 1000 radians/seconde et se trouve dans la plage d'environ 100 poises à environ 1000 poises à environ 1 radian/seconde,
d) suspendre ledit film entre ledit dispositif de revêtement et ledit substrat,
e) mettre en contact ledit film avec ledit substrat qui avance.

2. Procédé selon la revendication 1, dans lequel ladite composition thermoplastique comporte une composition adhésive thermofusible.

3. Procédé selon la revendication 2, comportant de plus la mise en suspension dudit film continu de sorte que ledit film augmente en force de cohésion afin que de quelconques fibres ou quelconques filaments présents dans le substrat ne pénètrent pas dans ledit film continu.

4. Procédé selon les revendications 1 à 3, dans lequel ledit substrat est choisi parmi le groupe constitué d'un matériau textile tissé, non tissé, de matériaux thermosensibles et de matières plastiques.

5. Procédé selon l'une quelconque des revendications 1 à 4, **caractérisé en ce que** ledit dispositif de revêtement est disposé à une distance par rapport audit substrat de préférence d'au moins environ 0,5 mm, et de manière plus préférée de pas plus d'environ 20 mm, et dans lequel le dispositif de revêtement est de préférence une buse fendue.

6. Procédé selon l'une quelconque des revendications 1 à 5, **caractérisé en ce que** le dispositif de revêtement est agencé dans une partie du trajet de déplacement du substrat, ledit trajet de déplacement étant dirigé essentiellement verticalement.

7. Procédé selon l'une quelconque des revendications 1 à 6, **caractérisé en ce que** la composition thermoplastique ou la composition adhésive thermofusible est revêtue sur le substrat de sorte que le poids de revêtement n'est pas supérieur à environ 30 g/m², de préférence pas plus d'environ 20 g/m² et de manière particulièrement préférée d'environ 10 g/m² ou moins.

8. Procédé selon l'une quelconque des revendications 1 à 7, **caractérisé en ce que** la composition thermoplastique ou la composition adhésive thermofusible est libérée du dispositif de revêtement à une température inférieure à environ 125°C, de préférence inférieure à environ 110°C.

9. Procédé selon l'une quelconque des revendications 1 à 7, dans lequel l'adhésif thermofusible est libéré du dispositif de revêtement à une température inférieure à 160°C.

10. Procédé pour produire un article hygiénique absorbant l'humidité comportant au moins un élément structurel de matériau absorbant, au moins une couche de barrière intérieure imperméable à l'humidité, dans ce cas perméable à la vapeur d'eau, recouvrant essentiellement l'élément absorbant et au moins une couche extérieure de matériau de textile qui est fixée à la couche de barrière sur une face, **caractérisé en ce que**, pour former la couche de barrière intérieure, une composition thermoplastique ou une composition adhésive thermofusible est revêtue selon l'une quelconque des revendications 1 à 9 sous forme d'un film fermé continu provenant d'un dispositif de revêtement sur le matériau textile de la couche extérieure sans mise en contact du dispositif de revêtement avec le substrat.

11. Procédé selon la revendication 10, **caractérisé en ce que** la composition thermoplastique, de préférence formée d'un film adhésif thermofusible, est revêtue sur le substrat en ayant un poids superficiel ne dépassant pas 30 g/m², de préférence n dépassant pas 20 g/m² et de manière particulièrement préférée inférieur à 10 g/m².

12. Procédé selon la revendication 10 ou 11, **caractérisé en ce que** la composition thermoplastique formant la couche de barrière est revêtue sur le matériau textile et ledit matériau est combiné après ceci au matériau formant l'élément absorbant de sorte que la composition thermofusible est collée uniquement au niveau des surfaces du matériau textile et de l'élément absorbant et ainsi relie le matériau textile et l'élément l'un à l'autre.

13. Procédé selon la revendication 12, **caractérisé en ce que** le matériau textile et l'élément absorbant sont combinés et reliés "en ligne" immédiatement après revêtement de la composition thermofusible.

14. Procédé de revêtement, dans lequel une composition thermoplastique ou une composition adhésive thermofusible qui a été rendue thermiquement apte à l'écoulement, est revêtue à partir d'un dispositif de revêtement sur un substrat, **caractérisé en ce que** ladite composition est libérée dudit dispositif de revêtement à une température inférieure à 125°C, de préférence inférieure à 110°C sous forme d'un revêtement continu sans contact entre ledit dispositif de revêtement et ledit substrat, ladite composition ayant une viscosité complexe inférieure à 500 poises à 1000 radians/seconde à la température de revêtement et une viscosité complexe dans la plage de 100 poises à 1000 poises à 1 radian/seconde à la température de revêtement, et est disposée par la suite sur la surface dudit substrat.

15. Article hygiénique jetable, comportant au moins une première couche, qui est un substrat perméable aux fluides corporels, et au moins une seconde couche qui est une couche de barrière imperméable aux fluides corporels formée par le procédé selon l'une quelconque des revendications 10 à 14.
